# FASCICULE DE BREVET EUROPEEN

(11) **EP 2 579 933 B1**
(45) Date de publication et mention de la délivrance du brevet: **21.05.2014**
(21) Numéro de dépôt: 11735469.6
(22) Date de dépôt: 09.06.2011
(51) Int. Cl.: A61M 15/00

(54) **DISPOSITIF DE DISTRIBUTION DE PRODUIT FLUIDE**
SPENDEVORRICHTUNG FÜR EIN FLUIDES PRODUKT
DEVICE FOR DISPENSING A FLUID PRODUCT

(30) Priorité: 11.06.2010 FR 1054634
(43) Date de publication de la demande: 17.04.2013
(73) Titulaire: Aptar France SAS, 27110 Le Neubourg (FR)
(72) Inventeur: PARDONGE, Jean-Marc, F-76520 Les Authieux Sur Port Saint Ouen (FR)
(74) Mandataire: CAPRI
(86) Numéro de dépôt international: PCT/FR2011/051314
(87) Numéro de publication internationale: WO 2011/154660

(56) Documents cités:
- EP-A1- 2 011 536
- WO-A1-2009/077697
- WO-A2-2008/081132
- FR-A1- 2 909 644
- FR-A1- 2 930 163

## Description

La présente invention concerne un dispositif de distribution de produit fluide, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à disposer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a chargé la dose (soit à partir d'un réservoir multidoses, soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. Un autre problème qui se pose avec les inhalateurs pourvus de bande de blisters est lié au déplacement de la bande, et au stockage de la partie utilisée de la bande. Ainsi, selon la longueur de la bande et/ou l'épaisseur des blisters, un espace important peut s'avérer nécessaire et tout blocage de la bande de blisters peut empêcher le bon fonctionnement de l'inhalateur. Par ailleurs, lorsque le dispositif d'avancée de la bande tire en même temps sur l'extrémité avant de la bande pour éviter un mauvais enroulement, il peut se poser un problème au fur et à mesure des actionnements en raison notamment du diamètre de la bande usée enroulée qui augmente progressivement. Les documents WO 2008/081132, FR 2930 163 A1, FR 2909 644 A1, EP 2011 536 et WO 2009/077697 décrivent des dispositifs de l'art antérieur.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un tel dispositif qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a aussi pour but de fournir un tel dispositif pourvu d'une bande de blisters, dans lequel le stockage de la partie de bande utilisée est optimisé, et le risque de blocage de la bande minimisé.

La présente invention a donc pour objet un dispositif de distribution de produit fluide, comportant un corps, ledit dispositif comportant en outre une bande souple allongée supportant une pluralité de réservoirs contenant chacun une dose de produit fluide ou pulvérulent, des moyens d'ouverture de réservoir pour ouvrir un réservoir respectif à chaque actionnement, des premiers moyens de déplacement pour faire avancer ladite bande souple avant et/ou pendant et/ou après chaque actionnement, pour amener un réservoir plein face auxdits moyens d'ouverture de réservoir et des seconds moyens de déplacement, pour déplacer un réservoir plein contre lesdits moyens d'ouverture à chaque actionnement du dispositif, l'extrémité avant de ladite bande souple, dans le sens d'avancement de celle-ci, étant fixée à un élément de réception rotatif, caractérisé en ce que ledit élément de réception comporte une denture périphérique coopérant avec un organe de traction mobile, un ressort chargé agissant sur ledit organe de traction mobile pour le déplacer, le déplacement dudit organe de traction mobile faisant tourner ledit élément de réception, de sorte que ledit élément de réception exerce une force de traction sur ladite bande allongée.

Avantageusement, ladite force de traction est maximale en début d'utilisation du dispositif et diminuant à chaque actionnement, au fur et à mesure que le ressort se décharge.

Avantageusement, ledit ressort est un ressort à traction, du type ressort à boudin.

Avantageusement, ledit élément de réception comporte une denture périphérique coopérant avec ledit organe de traction mobile.

Avantageusement, au moins une roue dentée est interposée entre ledit élément de réception et ledit organe de traction mobile.

Avantageusement, ledit ressort est fixé d'une part audit organe de traction mobile et d'autre part à une partie fixe du dispositif.

Avantageusement, ledit organe de traction mobile est un disque rotatif pourvu d'une denture périphérique coopérant avec la denture périphérique de l'élément de réception.

Avantageusement, ledit ressort est fixé audit disque rotatif à proximité de son bord périphérique.

Avantageusement, ledit organe de traction mobile est une tige crantée pourvue d'une denture coopérant avec la denture périphérique de l'élément de réception.

Avantageusement, ledit ressort est fixé à une extrémité de ladite tige.

Avantageusement, le déplacement de ladite tige est guidé par des moyens de guidage.

Avantageusement, lesdits moyens d'ouverture comportent une aiguille fixe par rapport audit corps, un réservoir étant déplacé contre ladite aiguille à chaque actionnement du dispositif, ladite aiguille pénétrant dans ledit réservoir pour le vider au moyen de l'écoulement d'inhalation.

Avantageusement, lesdits moyens d'ouverture sont commandés par l'inhalation de l'utilisateur, de sorte que le réservoir est simultanément ouvert et vidé par ledit écoulement d'inhalation.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- la figure 1 est une vue en section transversale d'un dispositif de distribution selon un premier mode de réalisation avantageux de l'invention,
- la figure 2 représente une vue schématique partielle en perspective du dispositif de la figure 1,
- la figure 3 est une vue similaire à celle de la figure 1 d'un second mode de réalisation avantageux de l'invention, et
- la figure 4 représente une vue schématique partielle en perspective du dispositif de la figure 3.

Sur les figures est représenté un exemple avantageux d'un inhalateur de poudre sèche. Cet inhalateur comporte un corps 10 sur lequel peuvent être montées coulissantes deux parties formant capot (non représentées) adaptées à être ouvertes pour ouvrir et charger le dispositif. Le corps 10 peut être de forme environ arrondie comme représenté sur les figures, mais il pourrait avoir tout autre forme appropriée. Le corps 10 comporte un embout buccal ou d'inhalation 1 définissant un orifice de distribution à travers lequel l'utilisateur va inhaler lors de l'actionnement du dispositif. Les capots peuvent s'ouvrir par pivotement autour d'un axe de rotation commun, mais tout autre moyen d'ouverture du dispositif est envisageable. En variante, le dispositif pourrait comporter un seul capot au lieu de deux.

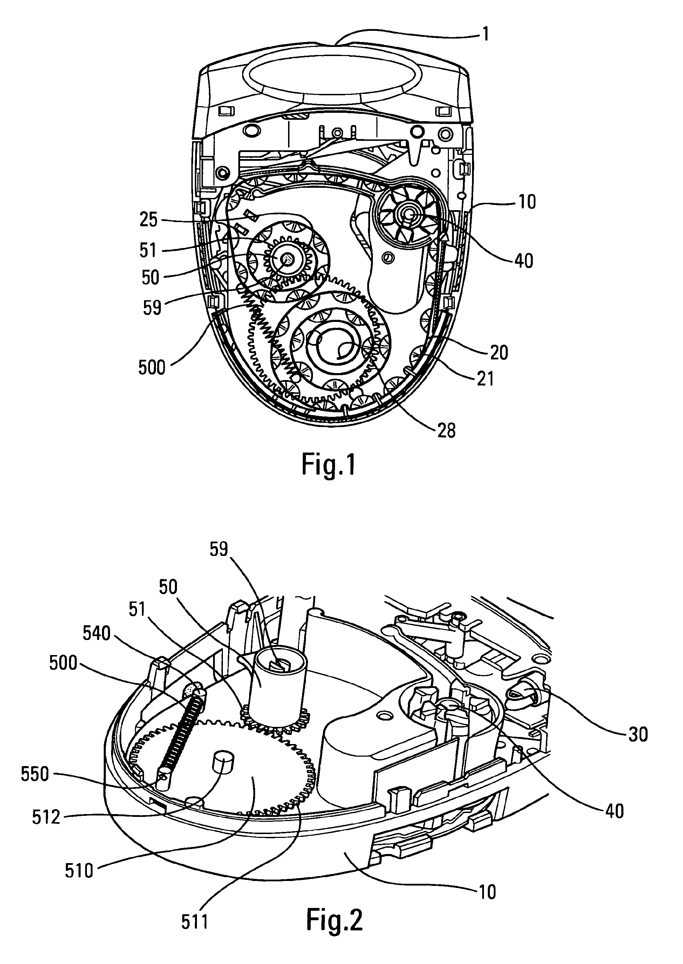

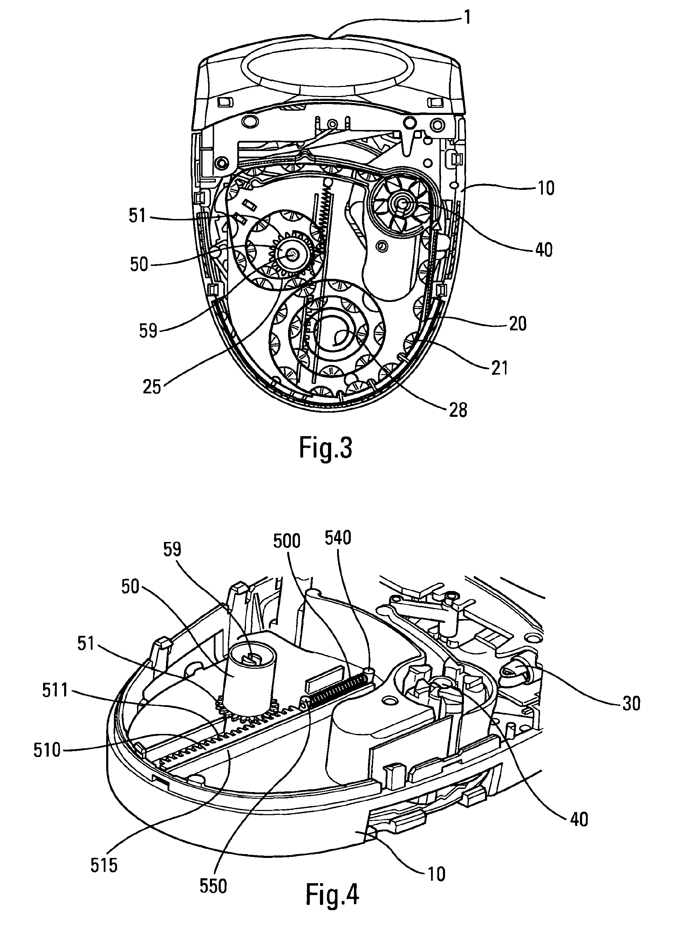

A l'intérieur du corps 10, il est prévu une bande 20 de réservoirs individuels 21, également appelés blisters, réalisée sous forme d'une bande allongée 20 sur laquelle les blisters 21 sont disposés les uns derrière les autres, de manière connue. Ces blisters 21, contenant de préférence de la poudre, ne sont pas représentés sur les figures 2 et 4 pour ne pas surcharger les dessin dans des buts de clarté. Cette bande de blister 20 est avantageusement constituée d'une couche ou paroi de base formant les cavités recevant les doses de poudre, et d'une couche ou paroi de fermeture qui obture de manière étanche chacun desdits blisters 21. Avant la première utilisation, la bande de blister 20 peut être enroulée à l'intérieur du corps 10, de préférence dans une partie de stockage, et des premiers moyens de déplacement de bande 40, notamment rotatifs, sont prévus pour progressivement dérouler et faire avancer cette bande de blister. Des seconds moyens de déplacement, notamment pivotants sur le corps 10, sont prévus pour amener un réservoir individuel ou blister respectif 21 dans une position de distribution à chaque actionnement du dispositif. La partie de bande comportant les réservoirs vides est avantageusement adaptée à s'enrouler dans un autre endroit dudit corps 10, de préférence une partie de réception, comme cela sera décrit plus en détail ci-après.

L'inhalateur comporte des moyens d'ouverture de réservoir 30 comportant de préférence des moyens de perçage et/ou de coupage de la couche de fermeture des blisters. Par exemple, les moyens d'ouverture de réservoir comportent avantageusement une aiguille 30, de préférence fixe par rapport au corps 10, et contre laquelle un blister respectif 21 est déplacé à chaque actionnement par les seconds moyens de déplacement. Le blister est alors percé par ladite aiguille, qui pénètre dans ledit blister pour expulser la poudre au moyen du flux d'inhalation de l'utilisateur.

Les premiers moyens de déplacement 40 sont adaptés à faire avancer la bande de blisters 20 avant et/ou pendant et/ou après chaque actionnement du dispositif. Les seconds moyens de déplacement sont adaptés à déplacer le réservoir à vider contre lesdits moyens de perçage et/ou de coupage 30 lors de l'actionnement. Ces seconds moyens de déplacement peuvent être sollicités par un élément élastique, tel qu'un ressort ou tout autre élément élastique équivalent, ledit élément élastique pouvant être préchargé lors de l'ouverture du dispositif. De préférence, les premiers moyens de déplacement comportent une roue d'indexage 40 qui reçoit et guide les blisters. Une rotation de cette roue 40 fait avancer la bande de blister 20. Dans une position angulaire particulière, un réservoir donné 21 est toujours en position face aux moyens d'ouverture 30. Les seconds moyens de déplacement peuvent comporter un élément de support pivotant autour d'un axe de rotation, ladite roue d'indexage 40 étant montée rotative sur ledit élément de support.

Un cycle d'actionnement du dispositif peut être le suivant. Lors de l'ouverture du dispositif, les deux parties latérales formant capot sont écartées l'une de l'autre en pivotant sur le corps pour ouvrir le dispositif, et ainsi charger le dispositif. Dans cette position la roue d'indexage 40 ne peut pas se déplacer vers l'aiguille 30 car les seconds moyens de déplacement sont retenus par des moyens de blocage appropriés. De préférence, c'est lors de l'inhalation par l'utilisateur à travers l'embout buccal 1 que ces moyens de blocage sont débloqués, ce qui provoque alors le déplacement de ladite roue d'indexage 40 en direction de l'aiguille 30, et donc l'ouverture d'un réservoir 21.

Dans les exemples représentés, le réservoir 21 se déplace vers sa position d'ouverture pour être ouvert par l'aiguille 30 qui est fixe par rapport au corps 10. Toutefois, il est envisageable que cette aiguille puisse également être mobile pendant la phase d'ouverture du réservoir 21. Par exemple, l'aiguille pourrait se déplacer en direction du réservoir 21 pendant que le réservoir 21 se déplace en direction de l'aiguille. Dans une autre variante, on pourrait également envisager que le réservoir 21 et l'aiguille se déplacent dans la même direction lors de l'actionnement, le réservoir 21 se déplaçant plus rapidement dans cette direction de sorte qu'il vient en contact avec l'aiguille pour être ouvert.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture de réservoir, on peut prévoir un système de déclenchement par l'inhalation qui comporte avantageusement une unité déplaçable et/ou déformable sous l'effet de l'inhalation (non représentée), cette unité étant adaptée à libérer les moyens de blocage. Cette unité comprend avantageusement une chambre d'air déformable. L'inhalation de l'utilisateur provoque la déformation de ladite chambre d'air déformable, permettant ainsi de libérer lesdits moyens de blocage et donc de permettre le déplacement des seconds moyens de déplacement, et donc d'un réservoir respectif 21, vers sa position d'ouverture. Le réservoir 21 n'est donc ouvert qu'au moment de l'inhalation, de sorte qu'il est simultanément vidé. Il n'y a donc aucun risque de perte de dose entre l'ouverture du réservoir et son vidage.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage des moyens de support mobiles et donc le déplacement du réservoir vers les moyens d'ouverture.

L'inhalateur comporte en outre une chambre de distribution ou dispersion (non représentée) qui est destinée à recevoir la dose de poudre après ouverture d'un réservoir respectif 21. Avantageusement, cette chambre de distribution est pourvue d'au moins une bille (non représentée) qui se déplace à l'intérieur de ladite chambre pendant l'inhalation, pour améliorer la distribution du mélange air et poudre après ouverture d'un réservoir 21, afin d'augmenter l'efficacité du dispositif.

Il peut être avantageux que les moyens d'ouverture 30, en particulier l'aiguille, soient formés directement sur ladite chambre de distribution, par exemple à l'extrémité d'un canal menant à ladite chambre.

Après l'inhalation, lorsque l'utilisateur referme le dispositif, tous les composants reviennent vers leurs positions de repos initiales. Le dispositif est alors prêt pour un nouveau cycle d'utilisation.

Selon un aspect avantageux de l'inhalateur, les réservoirs individuels ou blisters 21 sont formés sur une bande allongée 20, qui, au début, est principalement stockée sous forme de bobine dans un logement de stockage à l'intérieur du corps 10 du dispositif. Avantageusement, cette bande de blister enroulée 20 est maintenue par des parois internes dudit logement de stockage sans que son extrémité arrière 28 (dans le sens d'avancement de la bande de blisters 20) ne soit fixée par rapport audit corps 10, ce qui permet un assemblage plus aisé de cette bobine de bande de blister à l'intérieur du dispositif. La bande de blister 20 est déplacée par l'utilisateur avantageusement au moyen de la roue d'indexage 40 qui présente avantageusement au moins un, de préférence plusieurs évidements, dont la forme correspond à celle des blisters. Ainsi, lorsque cette roue d'indexage 40 tourne, elle fait avancer la bande de blister 20. Bien entendu en variante ou de manière additionnelle, on pourrait utiliser d'autres moyens d'avancée de bande de blisters, par exemple en prévoyant un profil sur les bords longitudinaux latéraux de la bande de blister ledit profil étant adapté à coopérer avec des moyens d'entraînement appropriés. De même, des trous ménagés le long des bords latéraux de la bande de blister pourraient également être utilisés pour faire avancer la bande de blister au moyen de roues dentées coopérant avec lesdits trous.

Après ouverture d'un ou plusieurs blister(s), la partie de bande de blister avec les réservoirs vidés doit pouvoir être stockée de manière aisée et non encombrante dans le dispositif, en évitant tout risque de blocage. Avantageusement, cette bande de blister usée s'enroule automatiquement sur elle-même pour à nouveau former une bobine.

Selon l'invention, l'extrémité avant 25 de cette bande de blister 20 est fixée à un élément de réception 50 monté rotatif. Pour assurer un bon enroulement de la partie avant de la bande de blisters 20, à savoir celle comportant les blisters vides, dans la partie de réception, cet élément de réception rotatif 50 est adapté à exercer une force de traction sur la bande 20, en particulier sur son extrémité avant 25. Ainsi, on évite tout risque de mauvais enroulement, par exemple de pliage en accordéon ou similaire, de la bande, qui risquerait de bloquer le dispositif. Cette force de traction est exercée par un ressort 500, de préférence un ressort à boudin, qui sollicite ledit élément de réception 50 en rotation, et donc tire sur la bande.

Les figures montrent des variantes de réalisation avantageuses, dans laquelle l'élément de réception 50 forme un cylindre monté rotatif autour d'un axe de rotation 59.

Selon l'invention, l'élément de réception rotatif 50 comporte une denture périphérique 51 coopérant avec un organe de traction mobile 510. Le ressort 500 est fixé d'une part audit organe de traction 510 en 550, et d'autre part à une partie fixe du dispositif en 540. Ainsi, le ressort exerce sa force de traction sur ledit organe de traction mobile 510 pour le déplacer, un déplacement dudit organe de traction mobile 510 provoquant une rotation de l'élément de réception 50, et donc une traction sur la partie avant 25 de la bande de blisters 20. Cette mise en oeuvre est avantageuse en ce qu'elle permet d'utiliser un ressort à traction classique, simple à assembler, contrairement à un ressort à spirale qui agirait directement sur l'élément de réception. La maitrise de la force de traction exercée par l'élément de réception 50 sur la bande est également améliorée.

Le ressort 500 est chargé avant la première utilisation ou lors de l'assemblage, et de manière évidente, la force de traction maximale exercée sur la bande 20 est insuffisante pour déchirer, déformer ou déplacer la bande 20 en l'absence d'actionnement. Progressivement, à chaque actionnement, le ressort se décharge en déplaçant l'organe de traction 510, ce qui fait tourner l'élément de réception 50. Les caractéristiques du ressort 500 sont de préférence choisies de telle sorte qu'il exerce une force de traction jusqu'aux dernières doses, mais dans certaines applications, il peut être suffisant que cette force de traction ne soit exercée qu'en début d'utilisation, pour garantir un bon début d'enroulement de la partie de bande avec les réservoirs vides.

Les figures 1 et 2 montrent un premier mode de réalisation, où l'organe de traction mobile 510 est un disque rotatif pourvu d'une denture périphérique 511 coopérant avec la denture 51 de l'élément de réception 50. Le ressort 500 est fixé à un plot 550 du disque rotatif 510 formé à proximité de son bord périphérique. Avantageusement, la dimension radiale du disque rotatif 510 est supérieure à celle de l'élément de réception pour fournir un nombre de dents maximal sur la denture périphérique 511 du disque 510. En effet, même en optimisant le positionnement du ressort 500, l'agencement des figures 1 et 2 permet un effet de déplacement du disque rotatif 510 sur moins d'un demi-tour. Eventuellement, on peut prévoir une (ou plusieurs) roue(s) dentée(s) supplémentaire(s) entre l'élément de réception 50 et l'organe de traction 510, pour démultiplier davantage la rotation dudit organe de traction 510.

Les figures 3 et 4 montrent un second mode de réalisation, où l'organe de traction mobile 510 est une tige crantée du type crémaillère. Les dents 511 de ladite tige 510 coopèrent avec la denture 51 de l'élément de réception 50. Dans cette variante, le ressort 500 est fixé à une extrémité 550 de ladite tige, et s'étend en prolongement longitudinal de celle-ci. Le ressort 500 chargé va donc tirer sur ladite tige 510, qui en se déplaçant, va faire tourner l'élément de réception 50. Avantageusement, des moyens de guidage 515 sont prévus pour guider le déplacement longitudinal de la tige 510. Des moyens de guidage similaires peuvent aussi être prévus pour le ressort 500.

D'autres types d'organe de traction sont aussi envisageables.

La force de traction exercée par l'élément rotatif 50 sur la bande 20 est totalement indépendante des premiers moyens de déplacement, à savoir la roue d'indexage 40 qui fait avancer la bande 20 lors de chaque actionnement. Ceci permet de garantir que la force de traction ne sera pas dépendante du diamètre d'enroulement de la bande de blisters usée, comme cela serait le cas si la rotation de l'élément rotatif de réception 50 était corrélée à celle de la roue d'indexage 40. Elle est aussi totalement indépendante des seconds moyens de déplacement, de sorte que l'invention évite de prévoir des moyens d'actionnement relativement complexes pour créer une force de traction sur la bande lors de l'actionnement de l'inhalateur. Ceci simplifie la fabrication et l'assemblage de l'inhalateur.

Avantageusement, l'élément de réception 50 est disposé environ au centre de la partie de réception. Celle-ci peut comporter des parois de guidage, en particulier une paroi de guidage externe, courbée, par exemple cylindrique, contre laquelle va glisser la bande de blisters 20. Il peut aussi y avoir une paroi de guidage interne prévue à l'entrée de la partie de réception, et s'étendant de préférence environ parallèlement à la paroi de guidage externe pour former un canal de guidage pour la bande de blisters 20. Ces parois de guidage favorisent encore davantage un bon enroulement de la bande de blisters 20 autour de l'élément de réception 50.

L'inhalateur de poudre sèche tel que décrit précédemment procure notamment les fonctions suivantes :
■ une pluralité de doses individuelles de poudre stockées dans des réservoirs individuels étanches, par exemple 30 ou 60 doses stockées sur une bande enroulée en bobine ;
■ la poudre libérée au moyen d'un perçage actionné par l'inhalation de l'utilisateur, ce perçage du blister étant réalisé au moyen d'un système de détection d'inhalation couplé à un système de libération préchargé ;
■ des moyens d'entraînement de forme appropriée en prises avec les blisters pour réaliser le déplacement de la bande de blister à chaque actionnement, et amener un nouveau réservoir dans une position dans laquelle il est destiné à être ouvert par les moyens d'ouverture appropriés ;
■ un stockage sûr et fiable de la partie de la bande usée, par enroulement autour d'un élément rotatif adapté à tirer sur la bande à chaque actionnement, cette traction étant totalement indépendante des premiers moyens de déplacement, à savoir de la roue d'indexage servant à faire avancer la bande de blisters.

D'autres fonctions sont également fournies par l'inhalateur tel que décrit précédemment. Il est à noter que les différentes fonctions, même si elles ont été représentées comme prévues simultanément sur les différents modes de réalisation de l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les réservoirs individuels sont arrangés les uns par rapport aux autres, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées.

## Revendications

1. Dispositif de distribution de produit fluide, comportant un corps (10), ledit dispositif comportant en outre une bande souple allongée (20) supportant une pluralité de réservoirs (21) contenant chacun une dose de produit fluide ou pulvérulent, des moyens d'ouverture de réservoir (30) pour ouvrir un réservoir respectif à chaque actionnement, des premiers moyens de déplacement (40) pour faire avancer ladite bande souple (20) avant et/ou pendant et/ou après chaque actionnement, pour amener un réservoir plein face auxdits moyens d'ouverture de réservoir et des seconds moyens de déplacement, pour déplacer un réservoir plein (21) contre lesdits moyens d'ouverture (30) à chaque actionnement du dispositif, l'extrémité avant (25) de ladite bande souple (20), dans le sens d'avancement de celle-ci, étant fixée à un élément de réception rotatif (50), **caractérisé en ce que** ledit élément de réception (50) comporte une denture périphérique (51) coopérant avec un organe de traction mobile (510), un ressort chargé (500) agissant sur ledit organe de traction mobile (510) pour le déplacer, le déplacement dudit organe de traction mobile (510) faisant tourner ledit élément de réception (50), de sorte que ledit élément de réception (50) exerce une force de traction sur ladite bande allongée (20).

2. Dispositif selon la revendication 1, dans lequel ladite force de traction est maximale en début d'utilisation du dispositif et diminuant à chaque actionnement, au fur et à mesure que le ressort (500) se décharge.

3. Dispositif selon la revendication 1 ou 2, dans lequel ledit ressort (500) est un ressort à traction, du type ressort à boudin.

4. Dispositif selon l'une quelconque des revendications précédentes, dans lequel ledit élément de réception (50) comporte une denture périphérique (51) coopérant avec ledit organe de traction mobile (510).

5. Dispositif selon l'une quelconque des revendications précédentes, dans lequel au moins une roue dentée est interposée entre ledit élément de réception (50) et ledit organe de traction mobile (510).

6. Dispositif selon l'une quelconque des revendications précédentes, dans lequel ledit ressort (500) est fixé d'une part audit organe de traction mobile (510) et d'autre part à une partie fixe du dispositif.

7. Dispositif selon l'une quelconque des revendications précédentes, dans lequel ledit organe de traction mobile (510) est un disque rotatif pourvu d'une denture périphérique (511) coopérant avec la denture périphérique (51) de l'élément de réception.

8. Dispositif selon la revendication 7, dans lequel ledit ressort (500) est fixé audit disque rotatif (510) à proximité de son bord périphérique.

9. Dispositif selon l'une quelconque des revendications 1 à 6, dans lequel ledit organe de traction mobile (510) est une tige crantée pourvue d'une denture (511) coopérant avec la denture périphérique (51) de l'élément de réception.

10. Dispositif selon la revendication 9, dans lequel ledit ressort (500) est fixé à une extrémité de ladite tige (510).

11. Dispositif selon la revendication 9 ou 10, dans lequel le déplacement de ladite tige (510) est guidé par des moyens de guidage (515).

12. Dispositif selon l'une quelconque des revendications précédentes, dans lequel lesdits moyens d'ouverture (30) comportent une aiguille (30) fixe par rapport audit corps (10), un réservoir (21) étant déplacé contre ladite aiguille à chaque actionnement du dispositif, ladite aiguille pénétrant dans ledit réservoir (21) pour le vider au moyen de l'écoulement d'inhalation.

13. Dispositif selon l'une quelconque des revendications précédentes, dans lequel lesdits moyens d'ouverture (30) sont commandés par l'inhalation de l'utilisateur, de sorte que le réservoir (21) est simultanément ouvert et vidé par ledit écoulement d'inhalation.

## Patentansprüche

1. Spendevorrichtung für ein fluides Produkt, das einen Körper (10) umfasst, wobei die genannte Vorrichtung des Weiteren über ein weiches, längliches Band (20) verfügt, das als Auflager für eine Vielzahl an Tanks (21) dient, die jeweils eine Dosis des flüssigen oder pulvrigen Produkts enthalten, Mittel zur Öffnung des Tanks (30), um einen entsprechenden Tank bei jedem Auslösen zu öffnen, erste Mittel zur Bewegung (40), damit das genannte weiche Band (20) bewegt wird, vor und/oder während und/oder nach jedem Auslösen, damit ein voller Tank vor die genannten Mittel zur Öffnung eines Tanks gebracht werden und zweite Mittel zur Bewegung, um einen vollen Tank (21) bei jedem Auslösen der Vorrichtung gegen die genannten Mittel zur Öffnung (30) zu bewegen, wobei das vordere Ende (25) des genannten weichen Bands (20) in der Richtung der Vorwärtsbewegung des Bands an einem drehenden Aufnahmesystem (50) befestigt ist, **dadurch gekennzeichnet, dass** das genannte Aufnahmeelement (50) über eine umfängliche Zahnung (51) verfügt, die mit einem beweglichen Zugorgan (510) zusammenarbeitet, um dies zu bewegen, wobei die Bewegung des genannten beweglichen Zugorgans (510) die Drehung des genannten Aufnahmeelements (50) bewirkt, sodass das genannte Aufnahmeelement (50) eine Zugkraft auf das genannte längliche Band (20) ausübt.

2. Vorrichtung nach Anspruch 1, bei der die genannte Zugkraft den Höchstwert bei Beginn der Verwendung der Vorrichtung erreicht und bei jedem Auslösen abnimmt, während die Feder (500) sich entlädt.

3. Vorrichtung nach Anspruch 1 oder 2, bei der die genannte Feder (500) eine Zugfeder vom Typ Schraubenfeder ist.

4. Vorrichtung nach einem der vorangegangenen Ansprüche, in der das genannte Aufnahmeelement (50) über eine umfängliche Zahnung (51) verfügt, die mit dem genannten beweglichen Zugorgan (510) zusammenarbeitet.

5. Vorrichtung nach einem der vorangegangenen Ansprüche, in der mindestens ein Zahnrad zwischen dem genannten Aufnahmeelement (50) und dem genannten beweglichen Zugorgan eingebaut wird.

6. Vorrichtung nach einem der vorangegangenen Ansprüche, in der die genannte Feder (500) auf der einen Seite an dem genannten beweglichen Zugelement (510) und auf der anderen Seite an einem festen Teil der Vorrichtung befestigt ist.

7. Vorrichtung nach einem der vorangegangenen Ansprüche, in der das genannte bewegliche Zugorgan (510) eine Drehscheibe ist, die über eine umfängliche Zahnung (511) verfügt, die mit der umfänglichen Zahnung (51) des Aufnahmeelements zusammenarbeitet.

8. Vorrichtung nach Anspruch 7, in der die genannte Feder (500) an der genannten Drehscheibe (510) nahe des umfänglichen Rands befestigt ist.

9. Vorrichtung nach einem der Ansprüche 1 bis 6, in der das genannte bewegliche Zugorgan (510) ein Stift mit Wellenschliff ist, der über eine Zahnung (511) verfügt, die mit der umfänglichen Zahnung (51) des Aufnahmeelements zusammenarbeitet.

10. Vorrichtung nach Anspruch 9, in der die genannte Feder (500) an einem Ende des genannten Stifts (510) befestigt wird.

11. Vorrichtung nach Anspruch 9 oder 10, in der die Bewegung des genannten Stifts (510) durch Führungsmittel (515) geführt werden.

12. Vorrichtung nach einem der vorangegangenen Ansprüche, in der die genannten Öffnungsmittel (30) über eine Nadel (30) verfügen, die fest zu dem besagten Körper (10) ist, einen Tank (21), der gegen die genannte Nadel bewegt wird, wenn die Vorrichtung ausgelöst wird, wobei die genannte Nadel in den genannten Tank (21) eindringt, um ihn mittels Inhalationsabfluss zu leeren.

13. Vorrichtung nach einem der vorangegangenen Ansprüche, in der die genannten Öffnungsmittel (30) durch Einatmen des Benutzers gesteuert werden, sodass der Tank (21) gleichzeitig durch den genannten Inhalationsabfluss geöffnet und geleert wird.

## Claims

1. A fluid dispenser device comprising a body (10), said device further comprising: an elongate flexible strip (20) supporting a plurality of reservoirs (21), each containing a dose of fluid or powder; reservoir-opening means (30) for opening a respective reservoir on each actuation; first displacement means (40) for causing said flexible strip (20) to advance before and/or during and/or after each actuation, so as to bring a full reservoir into register with said reservoir-opening means; and second displacement means for displacing a full reservoir (21) against said opening means (30) each time the device is actuated, the leading end (25) of said flexible strip (20), in the advance direction of said strip, being fastened to a rotary receiver element (50), said device being **characterized in that** said receiver element (50) includes a set of peripheral teeth (51) that co-operates with a movable traction member (510), a stressed spring (500) acting on said movable traction member (510) so as to displace it, the displacement of said movable traction member (510) causing said receiver element (50) to turn, such that said receiver element (50) exerts a traction force on said elongate strip (20).

2. A device according to claim 1, wherein said traction force is at a maximum when the device is first used and reduces on each actuation as the spring (500) relaxes.

3. A device according to claim 1 or claim 2, wherein said spring (500) is a traction spring of the coil spring type.

4. A device according to any preceding claim, wherein said receiver element (50) includes a peripheral set of teeth (51) that co-operates with said movable traction member (510).

5. A device according to any preceding claim, wherein at least one toothed wheel is interposed between said receiver element (50) and said movable traction member (510).

6. A device according to any preceding claim, wherein said spring (500) is fastened firstly to said movable traction member (510) and secondly to a stationary portion of the device.

7. A device according to any preceding claim, wherein said movable traction member (510) is a rotary disk that is provided with a peripheral set of teeth (511) that co-operates with the peripheral set of teeth (51) of the receiver element.

8. A device according to claim 7, wherein said spring (500) is fastened to said rotary disk (510) in the proximity of its peripheral edge.

9. A device according to any one of claims 1 to 6, wherein said movable traction member (510) is a notched rod that is provided with a set of teeth (511) that co-operates with the peripheral set of teeth (51) of the receiver element.

10. A device according to claim 9, wherein said spring (500) is fastened to one end of said rod (510).

11. A device according to claim 9 or claim 10, wherein the displacement of said rod (510) is guided by guide means (515).

12. A device according to any preceding claim, wherein said opening means (30) comprise a needle (30) that does not move relative to said body (10), a reservoir (21) being displaced against said needle each time the device is actuated, said needle penetrating into said reservoir (21) so as to empty it by means of an inhalation flow.

13. A device according to any preceding claim, wherein said opening means (30) are controlled by the user inhaling, such that the reservoir (21) is opened and emptied simultaneously by said inhalation flow.
